# EUROPÄISCHE PATENTSCHRIFT

(11) **EP 3 190 099 B1**
(45) Veröffentlichungstag und Bekanntmachung des Hinweises auf die Patenterteilung: **28.10.2020**
(21) Anmeldenummer: 16178475.6
(22) Anmeldetag: 07.07.2016
(51) Int. Cl.: C07C 57/03

(54) **NEUE FETTSÄURE UND IHRE VERWENDUNG**
NOVEL FATTY ACID AND THEIR USE
NOUVEL ACIDE GRAS ET SON UTILISATION

(30) Priorität: 14.09.2015 DE 102015115457; 14.09.2015 DE 102015012079; 14.09.2015 DE 102015012080; 04.05.2016 DE 102016108322
(43) Veröffentlichungstag der Anmeldung: 12.07.2017
(73) Patentinhaber: Helmholtz-Zentrum Potsdam - Deutsches GeoForschungsZentrum GFZ Stiftung des Öffentlichen Rechts des Lands Brandenburg, 14473 Potsdam (DE)
(72) Erfinder: Wagner, Dirk, 14469 Potsdam (DE); Mangelsdorf, Kai, 14469 Potsdam (DE)
(74) Vertreter: Hertin und Partner Rechts- und Patentanwälte PartG mbB

(56) Entgegenhaltungen:
- US-A1- 2008 039 525
- US-A1- 2014 274 968

## Beschreibung

Die Erfindung betrifft eine Fettsäure, ein Verfahren zur Herstellung der Fettsäure sowie Verwendungen der Fettsäure.

### HINTERGRUND

Fettsäuren sind von entscheidender Bedeutung für Zellen. So liefern sowohl gesättigte als auch ungesättigte Fettsäuren Energie, unterstützen das Immunsystem und wirken sich auf unterschiedliche Stoffwechselprozesse aus. Dabei korreliert ein hoher Anteil ungesättigter Fettsäuren in der Ernährung mit einem erhöhten Schutz vor unterschiedlichen Stoffwechselerkrankungen und bewirkt eine Senkung des Blutzucker- und Cholesterinspiegels. Insbesondere weisen Studien auf die positiven Wirkungen auf den menschlichen Körper von ungesättigten Omega-3 und Omega-6 Fettsäuren hin. So wurden für diese ungesättigten Fettsäuren unter anderem eine Verminderung des Risikos von Krebserkrankungen oder kardiovaskulären Entzündungskrankheiten sowie eine Verbesserung der Behandlung von Diabetes oder neurologischen Erkrankungen festgestellt (Gogus et. al., 2010). Obwohl die Wirkmechanismen der ungesättigten Fettsäuren in diesen Prozessen unterschiedlich sind, basiert eine zentrale Wirkung auf der Beteiligung der Fettsäuren am Aufbau der Zellmembran und einer Erhöhung der Fluidität der Membranen durch ungesättigte Fettsäuren. Ein Hauptbestandteil von Zellmembranen sind Phospholipide, welche aufgrund ihrer amphiphilen Eigenschaften am Aufbau einer Doppelmembran beteiligt sind. Grundsätzlich bestehen Phospholipide aus einer hydrophilen, polaren Kopfgruppe und zwei unpolaren, hydrophoben Fettsäuren. Die Kohlenstoffketten der Fettsäuren können gesättigt sein oder Doppelbindungen enthalten. Dabei sorgen gesättigte Fettsäuren und längere Kohlenstoffketten für eine starrere, geordnetere Struktur innerhalb der Membranen. Andererseits erhöhen ungesättigte Fettsäuren insbesondere mit einem hohen Anteil an cis-Doppelbindungen aufgrund von strukturellen "Knicken" in der Kohlenwasserstoffkette die Fluidität der Membran.

Aufgrund dieser positiven Eigenschaften haben ungesättigte Fettsäuren eine hohe Bedeutung in vielen wirtschaftlichen Bereichen erlangt. So werden ungesättigte Fettsäuren weitverbreitet in der Lebensmittelindustrie, der Tierernährung, der Kosmetik oder im Pharmabereich eingesetzt.

In der US20140274968 A1 wird ein Nahrungsergänzungsmittel umfassend eine Omega-3 Fettsäure offenbart, welche bevorzugt aus Krillöl gewonnen wird. Die Verabreichung des Nahrungsergänzungsmittels während der Adoleszenz soll die Gehirnleistung verbessern.

In der US20120064127 A1 werden ein medizinisch wirksames Nahrungsergänzungsmittel und eine pharmazeutische Komposition vorgeschlagen, welche die ungesättigten Fettsäuren Docosahexaensäure (DHA) und Eicosapentaensäure (EPA) umfassen, und zur Behandlung von Autoimmunkrankheiten, sowie neurologischen und gastroenterologischen Krankheiten eingesetzt wird.

In der WO2014092706 A1 wird ein medizinisch wirksames Nahrungsergänzungsmittel auf Basis einer Omega-3 Fettsäure vorgeschlagen, welche die Membranfluidität und die Aktivität von Peroxisom-Proliferator-aktivierte Rezeptoren erhöht und dadurch sich vorteilhaft auf die Gesundheit des menschlichen Körpers auswirkt.

In der DE102012007239 A1 wird eine pharmazeutische Komposition umfassend eine ungesättigte Fettsäure beschrieben, welche als antimikrobiell wirkende Zusammensetzung als Creme auf die Haut aufgetragen werden kann.

In dem US678451 wird eine enterale Ernährung mit Hilfe einer pharmazeutischen Komposition umfassend eine Docosahexaensäure (DHA) und Eicosapentaensäure (EPA) zur Behandlung von Patienten mit einer chronisch-entzündlichen Darmerkrankung beschrieben.

In der US20080039525 wird eine Lipidkomposition umfassend Omega-3, Omega-6 und Omega-9 Fettsäuren offenbart, welche durch bestimmte Verhältnisse von Omega-3, Omega-6 und Omega-9 Fettsäuren zueinander gekennzeichnet ist und als Nahrungsergänzungsmittel Diabetikern verabreicht wird. Die Verabreichung des Nahrungsergänzungsmittels soll insbesondere die Glukosetoleranz bei Diabetes-Patienten erhöhen und somit das Risiko vaskulärer Erkrankungen vermindern.

Weiterhin können die positiven Eigenschaften der ungesättigten Fettsäuren zur Erhöhung der Membranfluidität vorteilhaft in der Kryokonservierung von Zellen ausgenutzt werden.

In der WO2007149142 A1 ist ein Kryokonservierungsmedium beschrieben, welches die mehrfach ungesättigten Fettsäuren Docosahexaensäure (DHA) und Eicosapentaensäure (EPA) beinhaltet und zur Kryokonservierung von Zellen eingesetzt wird.

In dem US8071281 B2 wird ein Medium offenbart, welches Fettsäuren umfasst und das Wachstum, die Überlebensrate und letztendlich Lebendgeburtsrate von Embryonen und Oocyten erhöht, welche kryokonserviert wurden.

Die Effektivität der Verwendung der bekannten Fettsäuren zur Kryokonservierung sowie zur Behandlung von Erkrankungen oder zur Verbesserung des Zustandes von menschlichen und tierischen Organismen ist jedoch begrenzt und mit Nebenwirkungen verbunden.

Es war daher die Aufgabe der Erfindung eine neue Fettsäure bereitzustellen, die vorteilhafte Anwendungen ermöglicht und Nachteile bekannter Fettsäuren vermeidet. Ein Ziel war es eine ungesättigte Fettsäure herzustellen, die sich durch besonders positive Eigenschaften auf den menschlichen sowie tierischen Organismus auszeichnet. Insbesondere war es eine Aufgabe der Erfindung zu diesem Zweck eine ungesättigte Fettsäure herzustellen, welche zu einer besonders erhöhten Fluidität von Zellmembranen führt. Eine weitere Aufgabe der Erfindung war es eine Fettsäure herzustellen, welche sich aufgrund Ihrer Eigenschaften besonders gut für die Kryokonservierung von biologischem Material eignet.

### ZUSAMMENFASSUNG DER ERFINDUNG

Die Aufgabe wird durch eine Fettsäure gemäß dem unabhängigen Anspruch gelöst. Die abhängigen Ansprüche stellen bevorzugte Ausführungsformen der erfindungsgemäßen Fettsäure dar und beschreiben Verfahren zur Herstellung der Fettsäuren, sowie bevorzugte Verwendungen der Fettsäuren.

Die Erfindung betrifft somit eine Fettsäure gemäß Formel I: 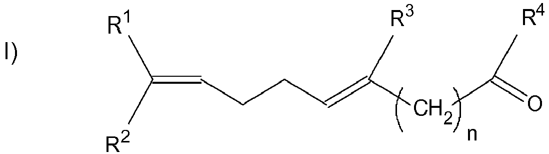 mit n größer gleich 4,
R¹ = (CH₂)ₖ-CH₃ mit k = 0, 1, 2 oder 3,
R² = (CH₂)ₘ-CH₃ mit m = 0, 1, 2, 3 oder 4,
R³ = Wasserstoff oder (CH₂)ₚ-CH₃ mit p = 0, 1, 2, 3 oder 4
   und
R⁴ = OH, SH, NH, Coenzym A (Thioester), Phosphatidylcholin, Phosphatidylethanolamin, Phosphatidylglycerol, Phosphatidylserin, Phosphatidylinositol, Sphingobase, substituiertes oder unsubstituiertes, ungesättigtes oder gesättigtes, verzweigtes oder unverzweigtes C₁-C₁₀-Alkyl, ein Rest der Formel II: 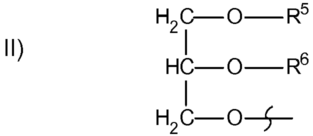 oder ein Rest der Formel III: 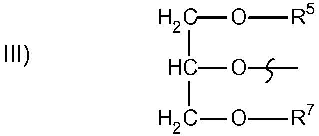
mit R⁵ = Wasserstoff, Phosphatidylcholin, Phosphatidylethanolamin, Phosphatidylglycerol, Phosphatidylserin, Phosphatidylinositol oder gesättigtes oder ungesättigtes C₂-C₂₄-Alkylcarbonyl,
R⁶ = Wasserstoff, gesättigtes oder ungesättigtes C₂-C₂₄-Alkylcarbonyl,
R⁷ = Wasserstoff, Phosphatidylcholin, Phosphatidylethanolamin, Phosphatidylglycerol, Phosphatidylserin, Phosphatidylinositol oder gesättigtes oder ungesättigtes C₂-C₂₄-Alkylcarbonyl,
Dabei zeigt die geschlängelte Linie 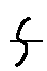 an, an welcher Stelle der Rest an die Verbindung bindet.

Die zwei durch zwei Methylengruppen unterbrochenen Doppelbindungen der erfindungsgemäßen Fettsäuren sorgen für besonders vorteilhafte Eigenschaften der erfindungsgemäßen Fettsäure. So können die zwei durch zwei Methylengruppen unterbrochenen Doppelbindungen überraschenderweise eine ringähnliche Struktur ausbilden, was dazu führt, dass die Fettsäure bei Integration in eine Membran die Membranfluidität erhöht. Dabei ist es besonders bevorzugt, dass die zwei durch zwei Methylengruppen unterbrochenen Doppelbindungen in der cis-Konfiguration vorliegen. Besonders bevorzugt ist, dass beide Doppelbindungen in der cis-Konfiguration vorliegen und besonders bevorzugt somit sowohl bei der ersten Doppelbindung der Rest R1 und ein Wasserstoffatom als auch bei der zweiten Doppelbindung der Rest R3 und ein Wasserstoffatom sich auf der gleichen Seite der Referenzebene befinden, die durch die Doppelbindung gegeben ist. In dieser Ausführungsform bildet die Fettsäure eine besonders stabile ringähnliche Struktur und es war völlig überraschend, dass gerade diese Struktur zu einer besonders erhöhten Fluidität einer Membran führt.

Neben einer cis/cis-Konfiguration haben sich aber auch andere Konfigurationen der Doppelbindungen als vorteilhaft erwiesen. Daher sind auch die Konfigurationen cis/trans, trans/cis und trans/trans bevorzugte Ausführungsformen der Erfindung.

Die bevorzugten Verbindungen für den Rest R4 erlauben dabei eine besonders effektive Integration der Fettsäure in eine Zellmembran und erhöhen somit die Biokompatibilität der genannten Fettsäure.

Es ist weiterhin bevorzugt, dass es sich bei der Fettsäure um eine Omega-3, Omega-4 oder Omega-6-Fettsäure handelt. So zeigt sich bei diesen bevorzugten Fettsäuren ein besonders positiver Effekt auf die Zellmembran in dem die Membranpermeabilität zur Aufnahme von Nährstoffen erhöht wird. Diese bevorzugten Fettsäuren sind besonders geeignet für die Therapie oder Prävention von kardiovaskulären Erkrankungen.

In einer bevorzugten Ausführungsform ist die Fettsäure dadurch gekennzeichnet, dass in der Formel I) k = 0, m = 1 und n= 4, 5, 6, 7, 8, 9 oder 10 ist. Dabei hat sich gezeigt, dass überraschenderweise ein Rest R1, welcher als Methylgruppe ausgebildet ist und ein Rest R2, welcher eine Ethylgruppe ist, die thermodynamische Stabilität der Formation einer ringähnlichen Struktur der zwei durch zwei Methylengruppen unterbrochenen Doppelbindungen erhöht. Weiterhin hat sich gezeigt, dass bevorzugte Ausführungsformen der Fettsäuren mit einer Kohlenstoffkettenlänge von 13 - 20 ein thermodynamisches Verhalten aufweisen, welches in einer Doppelmembran besonders effektiv den Phasenübergang der Membran zu niedrigeren Temperaturen absenkt. Dies war insbesondere im Hinblick auf die längeren Kohlenstoffketten überraschend, da bekannte längere Kohlenstoffketten mit einer starren Strukturanordnung in der Membran assoziiert sind. Durch diese Eigenschaften sind diese bevorzugten Fettsäuren besonders gut für die Kryokonservierung von biologischen Zellen, insbesondere humanen Zellen geeignet. Bei der Verwendung dieser bevorzugten Ausführungsform der erfindungsmäßen Fettsäure ist eine besonders hohe Überlebensrate, sowie anschließenden Wachstumsrate von humanen Zellen nach einer Kryokonservierung zu verzeichnen.

In einer besonders bevorzugten Ausführungsform ist die Fettsäure eine anteiso-Heptadeca-9,13-diensäure und in der Formel I) ist k = 0, m = 1, n = 7, R³ = Wasserstoff und R⁴ = OH. Alternativ wird diese besonders bevorzugte Fettsäure auch als eine 14-Methyl-Hexadec-9,13-diensäure oder eine anteiso-ω3,7-Heptadecadiensäure bezeichnet. Dabei ist es besonders bevorzugt, dass diese Fettsäure in der cis-Konfiguration vorliegt. Es hat sich gezeigt, dass die von den beiden Doppelbindungen der anteiso-Heptadeca-9,13-diensäure gebildete ringähnlichen Struktur, besonders vorteilhafte Auswirkungen auf die Membraneigenschaften von Zellen hat. So wird zum einen die Fluidität der Membran in einem besonders starken Maße erhöht und erlaubt einen effektiven Stoffwechsel der Zellen selbst bei Temperaturen nahe dem Gefrierpunkt. Hierbei erhöht die ringähnliche Struktur der bevorzugten Fettsäure insbesondere selektiv die Membranpermeabilität und erlaubt einen physiologischen Nährstoff- und lonenaustausch auch bei niedrigen Temperaturen. Die anteiso-Heptadeca-9,13-diensäure ist somit besonders gut für die Kryokonservierung von Zellen, Geweben oder Organismen geeignet. Darüber hinaus war es überraschend, dass die anteiso-Heptadeca-9,13-diensäure, und dabei insbesondere die cis,cis-Konfiguration, also die anteiso-Heptadeca-9c,13c-diensäure, besonders geeignet ist für die für präventive oder therapeutische Behandlung einer Vielzahl von Krankheiten. Die besonders bevorzugten Fettsäuren können vielfältig eingesetzt werden. Ein Einsatz dieser Fettsäuren ist beispielsweise bei Herzkreislauferkrankungen, Diabetes oder Fettleibigkeit möglich. Die bevorzugte Fettsäure kann bei der Verwendung besonders gut von den Patienten aufgenommen werden. Die Möglichkeit der besonders starken Erhöhung der Membranfluidität lässt einen Einsatz in der Kryokonservierung zu. Die Erhöhung der Membranfluidität kann aber auch Patienten mit Stoffwechselerkrankungen zugutekommen, wenn die bevorzugten Fettsäuren bei diesen Patienten eingesetzt werden. Die Eigenschaften der bevorzugten Fettsäure lassen es als Möglichkeit zu, dass diese den Blutzuckerspiegel bei Hyperglykämie reduzieren oder aber den Cholesterolspiegel. Bei einer Erkrankung des Fettstoffwechsels ist nicht nur ein Absenken des Gesamtcholesterinwertes erwünscht, sondern auch eine Verringerung des Anteils von Low Density Lipoprotein (LDL) und Erhöhung des Anteils von High Density Lipoproteine (HDL).

Offenbart wird auch eine pharmazeutische Zusammensetzung umfassend eine genannte Fettsäure, gegebenenfalls zusammen mit einem pharmazeutisch verträglichen Träger oder einem Verdünnungsmittel. Der Begriff pharmazeutische Zusammensetzung umfasst dabei bevorzugt eine applikationsfähige Mischung bestehend aus dem für die Verabreichung vorgesehenen Wirkstoff, der erfindungsgemäßen Fettsäuren oder bevorzugten Ausführungsformen davon, in einer höheren Dosis als der therapeutischen Mindestdosis. Dabei wird der pharmazeutisch verträgliche Träger bevorzugt an die Verabreichungsform angepasst. So kann der pharmazeutisch verträgliche Träger eine Trägerflüssigkeit sein für z.B. eine Injektions- oder Infusionslösung und unter Berücksichtigung der Applikationsart mit geeigneten Hilfsstoffen versetzt sein. Für eine orale Applikation kann der pharmazeutische Träger bevorzugt feste Füllstoffe umfassen, wie zum Beispiel Lactose, Zellulose, Saccharose oder Stärke. Für die Darreichung in einer Gelatinekapsel sind pharmazeutische Träger wie beispielsweise flüssige Polyethylenglycole oder Pflanzenöle besonders geeignet.

In einer weiteren bevorzugten Ausführungsform betrifft die Erfindung ein Verfahren zur Gewinnung der genannten Fettsäure durch Lipidextraktion von Bakterien der Familie *Flavobactericeae.* Bei Bakterien der Familie *Flavobactericeae* handelt es sich um gramnegative Bakterien. Besonders bevorzugt sind für die Durchführung des Verfahrens kryophile, psychrophile oder psychrotolerante Bakterien. Psychrophilie bezeichnet die Eigenschaft von Bakterien niedrige Temperaturen zu bevorzugen und kälteliebend zu sein. So können diese Bakterien bei Temperaturen unterhalb von 0°C leben, Stoffwechsel betreiben, wachsen und sich fortpflanzen. Kryophile Bakterien zeichnen sich durch eine Steigerung der Kälteanpassung aus und können ihren Stoffwechsel auch bei Temperaturen unter-10°C, -20°C, -25°C oder sogar-40°C aufrechterhalten. Psychrotolerante Bakterien sind ebenfalls an kalte Temperaturbedingungen angepasst, jedoch nicht im gleichen Ausmaß wie psychrophile oder kryophile Bakterien. So können psychotolerante Bakterien generell auch bei Temperaturen bis zum Gefrierpunkt wachsen, jedoch sind optimale Wachstumsbedingungen bei höheren Temperaturen von 15 - 20°C gegeben. Im Folgenden soll der Begriff psychrotolerante Bakterien, auch psychrophile oder kryophile Bakterien umfassen, außer es wird explizit auf Unterschiede zwischen den Graden der Kälteanpassung hingewiesen. Es wurde erfindungsgemäß erkannt, dass die Bakterien der Familie *Flavobactericeae,* insbesondere die psychrotoleranten Bakterien dieser Familie, sich besonders zur Gewinnung der erfindungsgemäßen Fettsäuren eignen. Dies könnte auf eine Anpassung ihrer Lebensbedingungen zurückzuführen sein, wodurch diese eine vielfältige Lipidkomposition der Membran beinhalten, welche ebenfalls die erfindungsgemäße Fettsäure in hinreichenden Konzentrationen umfasst.

Es ist besonders bevorzugt, dass das Verfahren dadurch gekennzeichnet ist, dass die Bakterien der Gattung *Chryseobacterium* angehören und bevorzugt *Chryseobacteria frigidisoli* und ganz bevorzugt *Chryseobacteria frigidisoli* des Stammes PB4^{T} sind.

*Chryseobacteria frigidisoli* ist ein psychrotolerantes, unbewegliches gram-negatives aerobes Bakterium, dass in sandigen antarktischen Permafrostböden vorkommt (Bajerski et al., 2013). Es war bisher nicht bekannt, dass diese Bakterien zu Herstellung der erfindungsgemäßen Fettsäuren verwendet werden können. Es hat sich jedoch gezeigt, dass die *Chryseobacteria frigidisoli* und insbesondere der Stamm PB4^{T} zum einen über eine besonders hohe Konzentration der genannten Fettsäure verfügen und diese besondere gut durch eine Lipidextraktion gewonnen werden kann. Für die Lipidextraktion eignen sich dabei bekannte Methoden des Standes der Technik zur Lipidextraktion von Fettsäuren aus Bakterien. Bevorzugt wird hierzu zunächst das Bakterium, bevorzugt das *Chryseobacteria frigidisoli* Bakterium besonders bevorzugt der Stamm PB4^{T} bereitgestellt. Chryseobakterien wie *Chryseobacterium greenlandense, antarctica* und *frigidisoli* lassen sich dabei insbesondere aus polaren Lebensräumen isolieren (Bajerski et al., 2013; Kämpfer et al., 2009a; Kämpfer et al., 2009b; Loveland-Curtze et al., 2010; Yi et al., 2005). Neben den polaren Lebensräumen lassen sich Chryseobakterien in vielen unterschiedlichen Umgebungen wie Böden (Weon et al., 2008; Zhou et al., 2007), Wasserreservoirs (Kim et al., 2008) und auch im klinischen Bereich (Kämpfer et al., 2009b) finden und isolieren. Der *Chryseobacterium frigidisoli* Stamm PB4^{T} lässt sich insbesondere aus sandig trockenen Permafrostablagerungen der Antarktis isolieren. Durch das kalte Klima mit sommerlichen Temperaturen von meist unter 0°C ist die Antarktis besonders geeignet um psychrophile und psychrotolerante Mikroben zur Gewinnung der erfindungsgemäßen Fettsäure zu finden. Diese Überlebenskünstler können den extremen klimatischen Bedingungen in arktischen und antarktischen Regionen standhalten (Bajerski et al., 2013; Loveland-Curtze et al., 2010; Yi et al., 2005). Um mit der Kälte fertig zu werden, ändern dabei die psychrophilen und psychrotoleranten Mikroorganismen die Lipidzusammensetzung ihrer Zellmembran. So kann die Fluidität und damit Funktionalität der Membranen aufrechterhalten werden (Mangelsdorf et al., 2009; Russell, 1989; Sinensky, 1974; Suutari und Laakso, 1994). Zu diesem Zweck verfügt insbesondere der *Chryseobacterium frigidisoli* Stamm PB4^{T} über die genannte Fettsäure und ist somit besonders für das Herstellungsverfahren geeignet.

Die Kultivierung der bereitgestellten *Flavobactericeae* wird bevorzugt an die Menge der zu gewinnenden Fettsäure angepasst. Verschiedene Verfahren zur Kultivierung der Bakterien können dabei zum Einsatz kommen. Für das besonders bevorzugte *Chryseobacterium frigidisoli* ist dabei eine Kultivierung auf Reasoner's 2A agar besonders geeignet und wird in Bajerski et al. (2013) detailliert beschrieben.

Es hat sich jedoch gezeigt, dass der Anteil der genannten Fettsäure sich bei verminderter Kultivierungstemperatur überraschenderweise erhöht. So wurden systematisch Kultivierungen von dem *Chryseobacterium frigidisoli* Stamm PB4^{T} bei Temperaturen zwischen 0°C und 30°C durchgeführt, wobei der relative Anteil der genannten Fettsäuren in der Lipidzusammensetzung der Membran bei niederen Temperaturen signifikant höher ist als bei höheren Temperaturen. Da die Wachstumsrate der *Chryseobacterium frigidisoli* bei ca. 18°C optimal ist, wird bevorzugt zur Herstellung der genannten Fettsäure eine Temperatur im Bereich von 5°C bis 15°C verwandt. Andere Kultivierungsmethoden sind ebenfalls geeignet und ein Fachmann wird diese entsprechend zu den kultivierenden Mikroorganismen anpassen.

Die Lipidextraktion der kultivierten Flavobakterien kann durch verschiedene Methoden der Lipidextraktion erfolgen, welche dem Fachmann aus dem Stand der Technik bekannt sind. Besonders geeignet ist eine Lipidextraktion unter Verwendung eines Durchflussmischsystem mit einem Verfahren nach Bligh and Dyer (1959). Die Fraktionierung der Lipide entsprechend ihrer Polarität in niedrig polare Lipide, Fettsäuren, Glycolipid und Phospholipide kann bevorzugt mit Verfahren durchgeführt werden, wie sie in Zink und Mangelsdorf (2004) beschrieben sind. Anschließend kann die genannte Fettsäure bevorzugt mit Hilfe der chromatographischer Methoden von den phospholipiden Fettsäuren isoliert werden.

Offenbart wird auch eine Verwendung der genannten Fettsäure oder einer genannten pharmazeutischen Zusammensetzung zur Verwendung als Medikament.

Es ist dabei besonders bevorzugt, dass das Medikament zur Behandlung von kardiovaskulären Krankheiten, Fettleibigkeit, Diabetes, Entzündungskrankheiten, Autoimmunkrankheiten und/oder neurologische Erkrankungen verwandt werden kann. Die Möglichkeit, dass die genannte Fettsäure zur Behandlung oder Prävention dieser Vielzahl von Krankheiten einsetzbar ist, war überraschend und für den Fachmann nicht naheliegend. Die erfindungsmäße Fettsäure und bevorzugte Ausführungsformen weisen die Möglichkeit auf, bei einer kontinuierlichen Verabreichung den Cholesterol- und Blutzuckerspiegel zu senken. Weiterhin besitzt die bevorzugte Fettsäure ein Potential zur Verringerung von Thromboxan und ist somit einsetzbar, um das Risiko von Thrombose zu erniedrigen. Darüber hinaus erlaubt die bevorzugte Fettsäure eine überraschende Möglichkeit zur Verbesserung der Signaltransduktion an den Membranen von Nervenzellen. Pathologische Signaltransduktionen sind die Ursache von einer Reihe verschiedener neurologischer Erkrankungen. Die bevorzugte Fettsäure ist daher weiterhin zur Behandlung und Prävention neurologischer Erkrankungen wie bevorzugt Depression, Angststörungen, Essstörungen, Schizophrenie und Alzheimer geeignet. Zudem lässt die erfindungsmäße Fettsäure und bevorzugte Ausführungsformen die Möglichkeit einer inhibierenden Wirkung auf Zytokine in Entzündungsreaktionen zu und ist dadurch bevorzugt einsetzbar zur Behandlung von Entzündungskrankheiten. Bevorzugt wird die Fettsäure zur Prävention und Therapie von chronisch entzündlichen Darmkrankheiten wie Colitis ulcerosa oder Morbus Chron, von rheumatoide Arthritis, Dermatitis oder Psoriasis verwandt.

Auch bevorzugt ist die Verwendung der Fettsäure oder der Zusammensetzung als Nahrungsergänzungsmittels bevorzugt zur Verbesserung der Konzentrationsfähigkeit, der Blutzirkulation und/oder des Wohlbefindens und/oder zur Verminderung von Schlaflosigkeit, Übelkeit und/oder Verdauungsproblemen einer Person. Dabei war es überraschend, dass die positiven Eigenschaften der erfindungsmäßen Fettsäure und bevorzugten Ausführungsformen davon als Nahrungsergänzungsmittel in einem breiten Spektrum das menschliche Wohlbefinden steigert. Hervorzuheben sind dabei insbesondere die Verbesserung der Blutzirkulation durch eine Reduzierung erhöhter Thrombozytenaktivität und Verbesserung der arteriellen Gefäße sowie die Erhöhung der Konzentrationsfähigkeit durch eine erhöhte Signaltransduktion an den Nervenzellen. Darüber hinaus wirkt sich die erfindungsgemäße Fettsäure bevorzugt positiv auf die Nährstoffaufnahme in den Darmzellen aus und verbessert dadurch die Verdauung bei einer kontinuierlichen Aufnahme des Nahrungsergänzungsmittels.

Weiterhin bevorzugt ist die Verwendung der Fettsäure oder der Zusammensetzung zur Erzielung einer kosmetischen Wirkung durch Auftragen auf die Haut, Haare oder Nägel einer Person. Die Fettsäure führt bei der kosmetischen Anwendung zu einem besseren Feuchtigkeitsgehalt der Haut, Haare und/oder Nägel, was vor allem im Winter von Vorteil ist. Durch die neuen Fettsäuren sind Haut, Haare und/oder Nägel in der Lage Feuchtigkeit auch bei niedrigen Temperaturen besser zu speichern. Es ist hierfür bevorzugt, dass die Fettsäure in Form einer kosmetischen Zusammensetzung verabreicht wird, z.B. als Creme, Öl, Lotion, Spray, Gel oder ähnlichem.

In einer weiteren bevorzugten Ausführungsform betrifft die Erfindung die Verwendung der Fettsäure oder der Zusammensetzung zur Kryokonservierung und/oder als Frostschutzmittel.

Es ist dabei bevorzugt, dass die Fettsäure oder die Zusammensetzung zur Kryokonservierung von tierischen, insbesondere menschlichen, Zellen insbesondere Stammzellen, Muskelzellen, Blutzellen, Nervenzellen, Hautzellen, Spermien und/oder Eizellen, von Geweben, insbesondere von Geweben des kardiovaskulären Systems, des Atmungssystems, des Verdauungssystems, des Harnwegsystems, des Bewegungsapparates und/oder des Nervensystems, von Embryonen und/oder von Organen insbesondere Herz, Niere, Darm, Lunge, Haut, Bauchspeicheldrüse und/oder Leber eingesetzt wird. Dabei war es völlig überraschend, dass bei Kryokonservierungsverfahren unter Verwendung der erfindungsgemäßen Fettsäuren die Möglichkeit gewährleistet wird, dass Zellen eine erhöhte Wachstumsrate aufweisen. Auch war es überraschend, dass sich die Überlebensrate von kryokonservierten Embryonen bevorzugt steigern lässt. Der Einbau von Doppelbindungen in die Zellmembran von Phospholipidfettsäuren ist ein entscheidender Prozess für alle Lebewesen. Überraschenderweise hat es sich gezeigt, dass der Anteil der erfindungsgemäßen Fettsäuren, vor allem der anteiso-heptadeca-9,13-diensäure mit abnehmenden Kultivierungstemperatur bei Mikroorganismen ansteigt. Dies stellt einen Anpassungsrestrukturierungsprozess dar, der die Zellmembran in einem fluideren Zustand hält. Die Mikroorganismen bringen ihre Membranphasenübergangstemperatur unter die Umgebungstemperatur durch Erhöhung des Anteils der erfindungsgemäßen Fettsäuren. Am wirksamsten hat sich dabei eine Steigerung von cis-konfigurierten Doppelbindungen erwiesen, weshalb die anteiso-heptadeca-9,13-diensäure der Erfindung bevorzugt in cis,cis-konfiguriert ist. Darüber hinaus war es überraschend, dass für die aus den Mikroorganismen isolierte Fettsäure diese positiven Eigenschaften in einer Vielzahl unterschiedlicher biologischen Materialien zur Geltung kommen und daher die Fettsäure besonders für die Kryokonservierung geeignet ist.

Weiterhin bevorzugt ist die Verwendung der Fettsäure oder der Zusammensetzung zur Kryokonservierung von prokaryotischen Zellen insbesondere von Bakterien und Archaeen, pflanzlichen Zellen und/oder pflanzlichen Geweben insbesondere Pflanzensamen, Knollen, Zwiebeln, Rhizome und/oder Keimlinge.

Offenbart wird auch eine Verwendung der Fettsäure oder der Zusammensetzung zur Differenzierung und Determination von tierischen Zellen und/oder pflanzlichen Geweben insbesondere von Stammzellen.

Offenbart wird auch die Verwendung der Fettsäure oder der Zusammensetzung zur Stabilisierung und Strukturverbesserung von industriell verarbeiteten Lebensmitteln insbesondere von Milchprodukten.

Es ist außerdem bevorzugt, dass die Fettsäure der Erfindung als Biomarker für Chryseobakterien verwendet wird. Besonders bevorzugt wird die Fettsäure als Biomarker für Chryseobacterium flavum, Chryseobacterium balustinum, Chryseobacterium gleum, Chryseobacterium indologenes, Chryseobacterium indoltheticum, Chryseobacterium joostei, Chryseobacterium scophthalmum, Chryseobacterium antarcticum und Chryseobacterium jeonii verwendet.

### SPEZIELLER BESCHREIBUNGSTEIL

Im Folgenden soll die Erfindung an Hand von Beispielen näher erläutert werden, ohne auf diese beschränkt zu sein.

### Beschreibung der Abbildungen

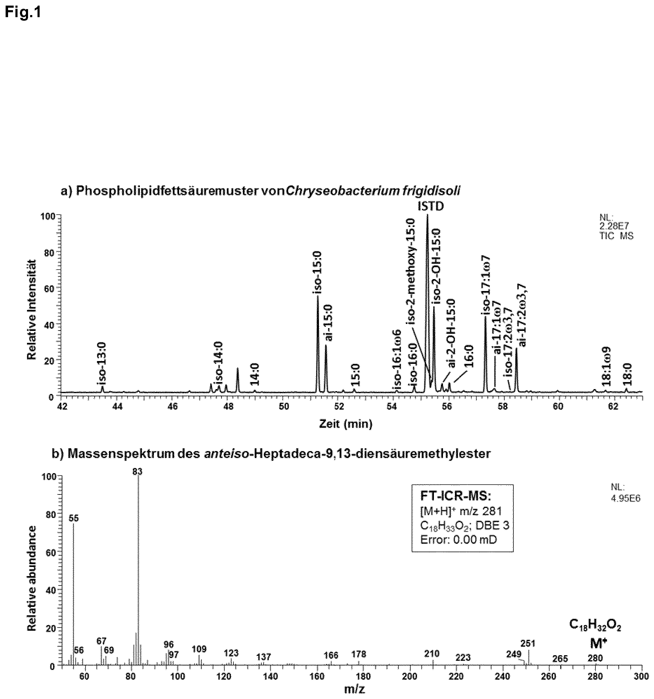

- Fig. 1: Chromatogramm einer Gaschromatographie Massenspektrometrie (GC-MS Chromatogramm) Messung der Phospholipidfettsäuren (PLFA) als Methylester der Zellmembran von *Chryseobacterium frigidisoli* bei einer Kultivierungstemperatur von 14°C. Die Abkürzungserklärungen sind in Fig. 7 aufgelistet. *Iso bezeichnet iso* verzweigte Fettsäuren und *ai anteiso-*verzweigte Fettsäuren. In der Standardnomenklatur für Fettsäuren entspricht X:Y der Anzahl der Kohlenstoffatome: Anzahl der Doppelbindungen; ω = bezeichnet die Position der Doppelbindung vom Ende der Kohlenstoffkette; ISTD = Interner Standard. b) Massenspektrum der *anteiso*-Heptadeca-9,13-diensäure (*ai*-17:2ω3,7) als Methylester. M⁺ = Molekülkation.

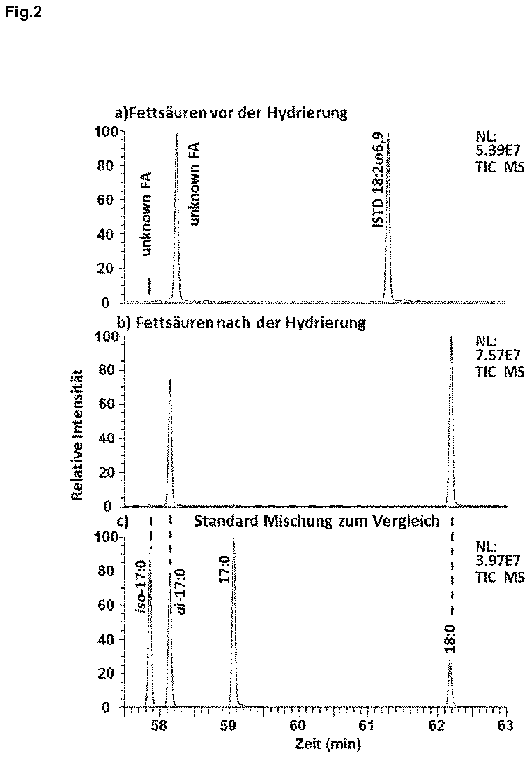

- Fig. 2: GC-MS Chromatogramm der über einen präparativen GC-Fraktionssammler isolierten *iso* und anteiso-Heptadeca-9,13-diensäure. Ein n-Octadeca-9,12-diensäuremethylesterstandard wurde hinzugegeben, um die erfolgreiche Hydrierung zu kontrollieren. b) GC-MS Chromatogramm *der iso-* und *anteiso-*Heptadeca-9,13-diensäuremethylester und des n-Octadeca-9,12-diensäuremethylesters nach der Hydrierung c) Zum Vergleich GC-MS-Chromatogramm der Standards *iso-* und *ai*-C_{17:0} sowie der *n*-C_{17:0} and *n*-C_{18:0} als Methylester.

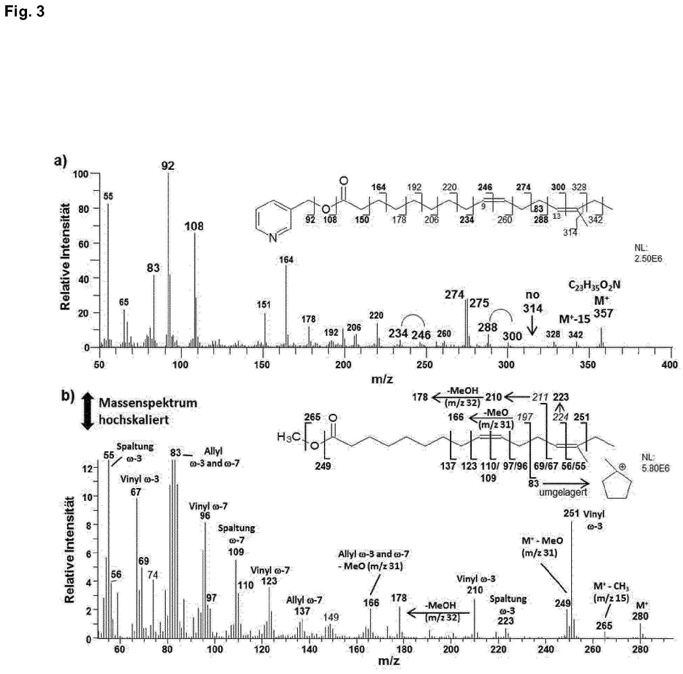

- Fig. 3: a) Massenspektrum und Fragmentinterpretation des 3-Pyridylcarbinolderivates der anteiso-Heptadeca-9,13-diensäure aus *Chryseobacterium frigidisoli. b)* Massenspektrum und Fragmentinterpretation des Methylesters der *anteiso-*Heptadeca-9,13-diensäure. Das Massenspektrum entspricht dem unter Abbildung 1, allerdings wurden hier zur besseren Sichtbarkeit die kleineren Massen hochskaliert. ω3 and ω7 = Doppelbindungen in Position 3 und 7 vom Ende der Kohlenstoffkette der Fettsäure aus gesehen. Allyl und vinyl = Spaltung in Allyl- (übernächste Bindung) oder Vinylposition (benachbarte Bindung) zu der Doppelbindung, M⁺= Molekülkation.

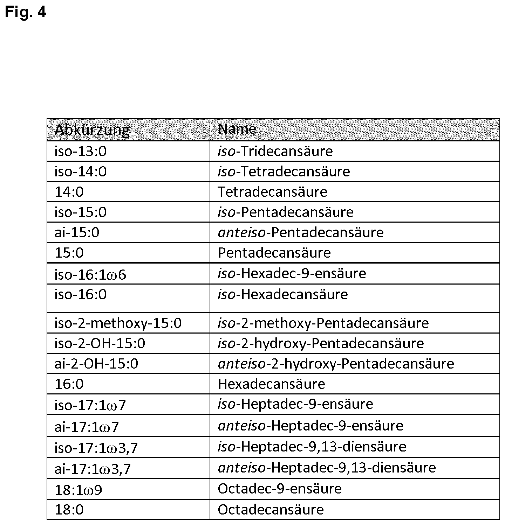

- Fig. 4: Abkürzungen sowie dazugehörige vollständige Bezeichnung der PLFA von Fig. 1. Es wird angemerkt, dass in Bajerski et al. (2013) die *iso*-17:1ω7 fehlerhafterweise als *iso*-17:1ω8 bezeichnet wurde.

### Beispiel 1:

### Isolierung und Kultivierung von Chryseobacterium frigidisoli

Zur Bereitstellung bevorzugter Flavobakterien zur Gewinnung der genannten Fettsäure wird das *Chryseobacterium frigidisoli* Stamm PB4^{T} von sandig trockenen Permafrostböden der Antarktis isoliert. Die Kultivierung des *Chryseobacterium frigidisoli* erfolgt wie in Bajerski et al. (2013) beschrieben, wobei die Temperaturspanne zwischen 0° und 30°C beträgt und bei niedrigeren Temperaturen einen höherer Anteil der der anteiso-Heptadeca-9,13-diensäure zu verzeichnen ist.

### Beispiel 2:

### Lipidextraktion von den Chryseobacterium frigidisoli

Die Lipide werden aus der mikrobiellen Kultur mit einem Verfahren nach Bligh and Dyer (1959) extrahiert. Die Fraktionierung der Lipide entsprechend ihrer Polarität in niedrig polare Lipide, Fettsäuren, Glycolipid and Phospholipide erfolgt mit Verfahrensschritten wie in Zink und Mangelsdorf (2004) beschrieben.

### Beispiel 3:

### Esterspaltung der Phospholipide und Methylierung de Phospholipidfettsäuren (PLFA)

Die phospholipide Fraktion aus Beispiel 2 wird in 50 µl Dichlormethan/Methanol (9:1 v/v) in einem 2 ml Fläschchen aufgelöst. Anschließend werden 50 µl von Trimethylsulfoniumhydroxid (TMSH) hinzugegeben und das Fläschchen wird verschlossen bevor es für 2 Stunden bei 70°C erhitzt wird (Mueller et al., 1990). Anschließend wird die Probe auf Raumtemperatur abgekühlt und mit Hilfe von Stickstoff getrocknet. Für die Gaschromotographie mit anschließender Massenspektrometrie (GS-MC) werden die methylierten Fettsäuren in 50 µl Dichlormethan (DMC) aufgelöst. Die erhaltenen Phospholipidfettsäuren (PLFA) der Zellmembran von *Chryseobacterium frigidisoli* werden anschließend mit einem GC-MS System gemessen (siehe Abb. 1 und Tab. 1).

### Beispiel 4:

### Trennung der anteiso-Heptadeca-9,13-diensäure mit Hilfe eines GC-Fraktionssammlers (PFC-System) und anschließende Hydrierung sowie GC-MS-Messungen der Produkte

Bevorzugte Ausführungsformen der Fettsäure werden von den PLFA des *Chryseobacterium frigidisoli* unter Verwendung eines Agilent 6890A PFC (Preparative Fraction Collector)-Systems der Firma Gerstel isoliert. Zum Nachweis, dass die Fettsäure verzweigt ist, erfolgt anschließend die Hydrierung der Fettsäuren mit Hilfe von Methanol, Adams Katalysator und Wasserstoff (siehe Fig. 2). Die Probe vor und nach der Hydrierung wird mit einem GC-MS System gemessen und mit einer Standardmischung verglichen (Fig. 2).

### Beispiel 5:

### Derivatisierung der anteiso-Heptadeca-9,13-diensäure zur Aufklärung der besonderen Struktureinheiten

100 µl Kaliumtertiärbutoxide wird in Tetrahydrofuran (1M) gelöst und mit 200 µl 3-Hydroxymethylpyridin versetzt. Nach Zugabe der PLFA Fraktion, gelöst in 1 ml Dichlormethan, wird die Mischung in einem verschlossenen Reagenzglas für 30 min auf 40 °C erhitzt. Nach dem Abkühlen auf Raumtemperatur werden 2 ml Wasser und 4 ml n-Hexane hinzugefügt. Nach der Phasentrennung wird die organische Phase abgetrennt und unter einem Stickstoffstrom eingedampft (Destaillats F et al., 2002). Für die GC-MS Messung werden die 3-Pyridylcarbinolesterderivate der PLFA von *Chryseobacterium frigidisoli* in 50µl Dichlormethan gelöst.

### Beispiel 6:

### GC-MS Analyse der PLFA Derivate zur Validierung der Struktur der anteiso-Heptadeca-9,13-diensäure

Zur Analyse der derivatisierten PLFA umfassend die besonders bevorzugte Ausführungsformen der Fettsäure (anteiso-Heptadeca-9,13-diensäure, *ai*-17:2ω3,7) wird bevorzugt ein gaschromatographisches System (Trace GC Ultra, Thermo Fisher Scientific Inc., Waltham, Massachusetts, USA) mit einem daran gekoppelten Massenspektrometer (DSQ , Thermo Fisher Scientific Inc., Waltham, Massachusetts, USA) verwendet.

In den Massenspektren der 3-Pyridylcarbinolesterderivate ist jede Kohlenstoff-Kohlenstoffbindung mit einem eigenen Fragment gekennzeichnet (Abb. 3a). Aus diesem Grund zeigt eine gesättigte Fettsäure Hauptfragmente, die sich jeweils um 14 Masseneinheiten entsprechend einer CH₂-Gruppe unterscheiden. Doppelbindungen sind durch einen Unterschied von 12 Massen angezeigt, wobei die Doppelbindungsposition dann zwischen dem Kohlenstoffatom, das den Massenzuwachs von nur 12 Massen zu verzeichnen hat, und dem nächsten Kohlenstoffatom lokalisiert ist (ausgehend von der funktionellen Gruppe). Des Weiteren sind Verzweigungen in der Struktur durch die Abwesenheit des entsprechenden Fragmentes gekennzeichnet. Wie in der Massenspektrometrie üblich bezeichnet m/z den Masse-Ladungs-Quotienten.
Die prominenten Fragmente m/z 92 und 108 im Massenspektrum des 3-Pyridylcarbinolesterderivates der anteiso-Heptadeca-9,13-diensäure entstammen dem Kopfgruppenteil des Derivates (Abb. 3a). Die Fragmente m/z 151 (m/z 150+H) und 164 stellen die ersten regulären Fragmente der aliphatischen Kette ausgehend von der funktionellen Gruppe dar. Doppelbindungen werden durch einen Massenzuwachs von 12 Massen zwischen m/z 234 und 246 sowie zwischen m/z 288 und 300 angezeigt. Dies entspricht den Doppelbindungspositionen Δ⁹ und Δ¹³. Das Allylfragment zwischen beiden bismethylen unterbrochenen Doppelbindungen ist sehr prägnant (m/z 274/275). Das Gegenfragment ist m/z 83. Die *anteiso*-Verzweigung, die auch im Hydrierungsexperiment (Abb. 2) angezeigt ist, spiegelt sich durch das Fehlen des entsprechenden Fragments (m/z 314) wider. Zusammen mit dem Hydrierungsexperiment belegt das 3-Pyridylcarbinolesterderivat, dass es sich bei der besonderen Fettsäure um 14-methyl-Hexadeca-9,13-diensäure (anteiso-Heptadeca-9,13-diensäure, *ai*-17:2Δ^{9,13} oder auch *anteiso*-ω3,7-Heptadecadiensäure, *ai*-17:2ω3,7; *ai*-17:2n-3,7) handelt.
Das Massenspektrum des *anteiso*-Heptadeca-9,13-diensäuremethylesters (*ai*-17:2ω3,7) wird, wie in Abb. 1b gezeigt, vom m/z 83 Peak dominiert. Aus diesem Grund wird in Abb. 3 das Massenspektrum des anteiso-Heptadeca-9,13-diensäuremethylesters hochskaliert, um dadurch die Sichtbarkeit der kleineren Fragmente zu erhöhen. Das Molekülkation wird in Abb. 1b und 3b durch m/z 280 repräsentiert, m/z 265 zeigt den Verlust der terminalen Estermethylgruppe an (M⁺-15) und m/z 249 den Verlust einer Methoxygruppe (M⁺-31). m/z 251 entspricht dem Vinylfragment des Kettenendes der ω3-Doppelbindung (der Doppelbindung zwischen dem 13 und 14 Kohlenstoffatom). Die Spaltung der ω3-Doppelbindung wird durch m/z 223 (m/z 224-H) repräsentiert, das Gegenfragment stellt m/z 56 bzw. 55 (-H) dar. Darüber hinaus sind die Fragmente m/z 251 und m/z 223 prominente Fragmente für *anteiso*-Verzweigungen (M⁺-29 sowie M⁺-57). m/z 210 (m/z 211-H) und m/z 178 (-MeOH, m/z 32) bilden die weiteren Vinylfragmente der ω3-Doppelbindung mit den zugehörigen Gegenfragmenten des Kettenendes m/z 69 and 67 (-2H). Das Fragment an m/z 166 repräsentiert das Allylfragment nach dem Verlust einer Methoxygruppe (-MeO, m/z 31). m/z 109 repräsentiert das Kettenendfragment der ω7-Doppelbindung und m/z 97 und m/z 96 (-H) sowie m/z 123 bilden das Vinylfragment des Kettenendes der ω7-Doppelbindung. m/z 137 ist das Allylfragment des Kettenendes der ω7-Doppelbindung.

Es wird darauf hingewiesen, dass verschiedene Alternativen zu den beschriebenen Ausführungsformen der Erfindung verwendet werden können, um die Erfindung auszuführen und zu der erfindungsgemäßen Lösung zu gelangen. Die erfindungsgemäße Fettsäure, sowie die Verfahren zur Herstellung oder Verwendung derselben beschränken sich in ihren Ausführungen somit nicht auf die vorstehenden bevorzugten Ausführungsformen. Vielmehr ist eine Vielzahl von Ausgestaltungsvarianten denkbar, welche von der dargestellten Lösung abweichen können. Ziel der Ansprüche ist es, den Schutzumfang der Erfindung zu definieren. Der Schutzumfang der Ansprüche ist darauf gerichtet, die erfindungsgemäße Fettsäuren, Verfahren zur Herstellung der Fettsäure und Verwendungen der Fettsäure sowie äquivalente Ausführungsformen von diesen abzudecken.

### LITERATURVERZEICHNIS

Bajerski, F., Ganzert, L., Mangelsdorf, K., Padur, L., Lipski, A., Wagner, D., 2013. Chryseobacterium frigidisoli sp. nov., a psychrotolerant species of the family Flavobacteriaceae isolated from sandy permafrost from a glacier forefield. International Journal of Systematic and Evolutionary Microbiology 63, 2666-2671.
Bligh, E.G., Dyer, W.J., 1959. A rapid method of total lipid extraction and purification. Canadian Journal of Biochemistry and Physiology 37, 911-917.
Destaillats F, Angers P. 2002. On-step methodology for the synthesis of FA picolinyl esters from intact lipids. Journal of the American Oil Chemists' Society 79,253-256.
Gogus, U. und Smith, C., 2010, n-3 Omega fatty acids: a review of current knowledge. International Journal of Food Science and Technology 45, 417-436.
Kämpfer, P., Lodders, N., Vaneechoutte, M., Wauters, G., 2009a. Transfer of Sejongia antarctica, Sejongia jeonii and Sejongia marina to the genus Chryseobacterium as Chryseobacterium antarcticum comb. nov., Chryseobacteriumjeonii comb. nov. and Chryseobacterium marinum comb. nov. International Journal od Systematic and Evolutionary Microbiology 59, 2238-2240.
Kämpfer, P., Vaneechoutte, M., Lodders, N., De Baere, T., Avesani, V., Janssens, M., Busse, H.-J., Wauters, G., 2009b. Description of Chryseobacterium anthropi sp. nov. to accomodate clinical isolates biochemically similar to Kaistella koreensis and Chryseobacterium haifense proposal to reclassify Kaistella koreense comb. nov. and emended description of the genus Chryseobacterium. International Journal of Systematic and Evolutionary Microbiology 59, 2421-2428.
Kim, K.K., Lee, K.C., Oh, H.-M., Lee, J.-S., 2008. Chryseobacterium aquaticum sp. nov. isolated from water reservoir. International Journal of Systematic and Evolutionary Microbiology 58, 533-537.
Loveland-Curtze, J., Miteva, V., Brenchley, J., 2010. Novel ultramicrobacterial isolates from a deep Greenland ice core represent a proposed new spezies, Chryseobacterium greenlandense sp. nov. Extremophiles 14, 61-69.
Mangelsdorf, K., Finsel, E., Liebner, S., Wagner, D., 2009. Temperature adaptation of microbial communities in different horizons of Siberian permafrost-affected soils from the Lena-Delta. Chemie der Erde 69, 169-182.
Mueller, K.D., Husmann, H., Nalik, H.P., 1990. A new and rapid method for the assay of bacterial fatty acids using high resolution capillary gas chromatography and trimethylsulfonium hydroxide. Zentralblatt für Bakteriologie: International journal of medical microbiology 274, 174-182.
Russell, N.J., 1989. Functions of lipids: Structural roles and membrane functions, in: Ratledge, C., Wilkinson, S.G. (Eds.), Microbial lipids 2. Academic Press, London, pp. 279-365.
Sinensky, M., 1974. Homeoviscous Adaptation - A homeostatic process that regulates the viscosity of membrane lipids in Escherichia coli. Proc. Natl. Acad. Sci. USA 71, 522-525.
Suutari, M., Laakso, S., 1994. Microbial fatty acids and thermal adaptation. Critical Reviews in Microbiology 20, 285-328.
Weon, H.Y., Kim, B.-Y., Yoo, S.-H., Kwon, S.-W., Stackebrandt, E., Go, S.-J., 2008. Chryseobacterium soli sp. nov. and Chryseobacterium jejuense sp. nov. isolated from soil samples from Jeju, Korea. International Journal of Systematic and Evolutionary Microbiology 58, 470-473.
White, D.C., Davis, W.M., Nickels, J.S., King, J.D., Bobbie, R.J., 1979. Determination of the sedimentary microbial biomass by extractible liquid phosphate. Oecologia 40, 51-62. Yi, H., Yoon, H.I., Chun, J., 2005. Sejongia antarctica gen. nov., sp. nov. and Sejongia jeonii sp. nov., isolated from the Antarctica. International Journal of Systematic and Evolutionary Microbiology 55, 409-416.
Zhou, Y., Dong, J., Wang, X., Huang, X., Zhang, K.-Y., Zhang, Y.-Q., Guo, Y.-F., Lai, R., Li, W.-J., 2007. Chryseobacterium flavum sp. nov.., isolated from polluted soil. International Journal of Systematic and Evolutionary Microbiology 57, 1765-1769.
Zink, K.-G., Mangelsdorf, K., 2004. Efficient and rapid method for extraction of intact phospholipids from sediments combined with molecular structure elucidation using LC-ESI-MS-MS analysis. Analytical and Bioanalytical Chemistry 380, 798-812.

## Patentansprüche

1. Fettsäure gemäß Formel I: 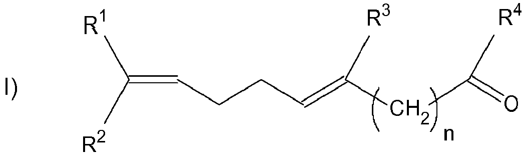 mit n größer gleich 4,
R¹ = (CH₂)ₖ-CH₃ mit k = 0, 1, 2 oder 3,
R² = (CH₂)ₘ-CH₃ mit m = 0, 1, 2, 3 oder 4,
R³ = Wasserstoff oder (CH₂)ₚ-CH₃ mit p = 0, 1, 2, 3 oder 4
und
R⁴ = OH, SH, NH, Coenzym A (Thioester), Phosphatidylcholin, Phosphatidylethanolamin, Phosphatidylglycerol, Phosphatidylserin, Phosphatidylinositol, Sphingobase, substituiertes oder unsubstituiertes, ungesättigtes oder gesättigtes, verzweigtes oder unverzweigtes C₁-C₁₀-Alkyl, ein Rest der Formel II: 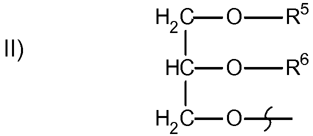 oder ein Rest der Formel III: 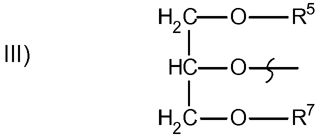
mit R⁵ = Wasserstoff, Phosphatidylcholin, Phosphatidylethanolamin, Phosphatidylglycerol, Phosphatidylserin, Phosphatidylinositol oder gesättigtes oder ungesättigtes C₂-C₂₄-Alkylcarbonyl,
R⁶ = Wasserstoff, gesättigtes oder ungesättigtes C₂-C₂₄-Alkylcarbonyl,
R⁷ = Wasserstoff, Phosphatidylcholin, Phosphatidylethanolamin, Phosphatidylglycerol, Phosphatidylserin, Phosphatidylinositol oder gesättigtes oder ungesättigtes C₂-C₂₄-Alkylcarbonyl.

2. Fettsäure gemäß Anspruch 1 **dadurch gekennzeichnet, dass** die zwei durch zwei Methylengruppen unterbrochenen Doppelbindungen in der cis-Konfiguration vorliegen.

3. Fettsäure gemäß Anspruch 1 oder 2 **dadurch gekennzeichnet, dass** in der Formel I) k = 0, m = 1 und n= 4, 5, 6, 7, 8, 9 oder 10 ist.

4. Fettsäure gemäß einer der vorherigen Ansprüche **dadurch gekennzeichnet, dass** die Fettsäure eine anteiso-Heptadeca-9,13-diensäure ist und in der Formel I) k = 0, m = 1, n = 7, R³ = Wasserstoff und R⁴ = OH ist.

5. Verfahren zur Gewinnung einer Fettsäure gemäß einem der Ansprüche **1 - 4** durch Lipidextraktion von Bakterien der Familie *Flavobactericeae.*

6. Verfahren zur Gewinnung einer Fettsäure nach dem vorherigen Anspruch **dadurch gekennzeichnet, dass** die Bakterien der Gattung *Chryseobacterium* angehören und bevorzugt *Chryseobacteria frigidisoli* und ganz bevorzugt *Chryseobacteria frigidisoli* des Stammes PB4^{T} sind.

7. Verwendung einer Fettsäure gemäß einem der Ansprüche 1 - 4 als Nahrungsergänzungsmittels bevorzugt zur Verbesserung der Konzentrationsfähigkeit, der Blutzirkulation und/oder des Wohlbefindens und/oder zur Verminderung von Schlaflosigkeit, Übelkeit und/oder Verdauungsproblemen einer Person und/oder als kosmetisches Mittel bevorzugt zur Erzielung einer kosmetischen Wirkung durch Auftragen auf die Haut, Haare oder Nägel einer Person.

8. Verwendung einer Fettsäure gemäß einem der Ansprüche **1 - 4** zur Kryokonservierung und/oder als Frostschutzmittel.

9. Verwendung einer Fettsäure gemäß einem der Ansprüche **1 - 4** zur Kryokonservierung von tierischen, insbesondere menschlichen, Zellen insbesondere Stammzellen, Muskelzellen, Blutzellen, Nervenzellen, Hautzellen, Spermien und/oder Eizellen, von Geweben, insbesondere von Geweben des kardiovaskulären Systems, des Atmungssystems, des Verdauungssystems, des Harnwegsystems, des Bewegungsapparates und/oder des Nervensystems, von Embryonen und/oder von Organen insbesondere Herz, Niere, Darm, Lunge, Haut, Bauchspeicheldrüse und/oder Leber.

10. Verwendung einer Fettsäure gemäß einem der Ansprüche **1 - 4** zur Kryokonservierung von prokaryotischen Zellen insbesondere von Bakterien und Archaeen, pflanzlichen Zellen und/oder pflanzlichen Geweben insbesondere Pflanzensamen, Knollen, Zwiebeln, Rhizome und/oder Keimlinge.

## Claims

1. A fatty acid according to formula I: 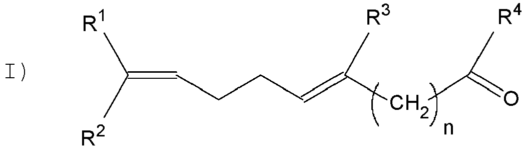
where n is greater than or equal to 4,
R¹ = (CH₂)ₖ-CH₃ where k = 0, 1, 2 or 3,
R² = (CH₂)ₘ-CH₃ where m = 0, 1, 2, 3 or 4,
R³ = hydrogen or (CH₂)ₚ-CH₃ where p = 0, 1, 2, 3 or 4
and
R⁴ = OH, SH, NH, coenzyme A (thioester), phosphatidylcholine, phosphatidylethanolamine, phosphatidylglycerol, phosphatidylserine, phosphatidylinositol, sphingobases, substituted or unsubstituted, unsaturated or saturated, branched or unbranched C₁-C₁₀-alkyl, a residue of formula II: 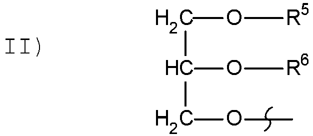 or a residue of formula III: 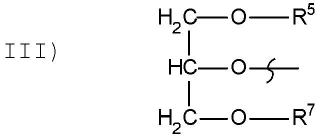
where R⁵ = hydrogen, phosphatidylcholine, phosphatidylethanolamine, phosphatidylglycerol, phosphatidylserine, phosphatidylinositol, or saturated or unsaturated C₂-C₂₄-alkylcarbonyl,
R⁶ = hydrogen, saturated or unsaturated C₂-C₂₄-alkylcarbonyl,
R⁷ = hydrogen, phosphatidylcholine, phosphatidylethanolamine, phosphatidylglycerol, phosphatidylserine, phosphatidylinositol, or saturated or unsaturated C₂-C₂₄-alkylcarbonyl.

2. The fatty acid according to claim 1, **characterized in that** the two double bonds interrupted by two methylene groups are in the cis-configuration.

3. The fatty acid according to claim 1 or 2, **characterized in that** in formula I), k = 0, m = 1, and n = 4, 5, 6, 7, 8, 9 or 10.

4. The fatty acid according to any one of the preceding claims, **characterized in that** the fatty acid is an anteiso-heptadeca-9,13-dienoic acid, and in formula I), k = 0, m = 1, n = 7, R³ = hydrogen, and R⁴ = OH.

5. A method for obtaining a fatty acid according to any one of claims 1 - 4 by lipid extraction of bacteria from the family *Flavobacteriaceae.*

6. The method for obtaining a fatty acid according to the preceding claim, **characterized in that** the bacteria belong to the genus *Chryseobacterium* and are preferably *Chryseobacteria frigidisoli* and very preferably *Chryseobacteria frigidisoli* of the PB4^{T} strain.

7. A use of a fatty acid according to any one of claims 1-4 as a dietary supplement, preferably to improve a person's concentration, circulation, and/or overall wellness, and/or to alleviate insomnia, nausea, and/or digestive problems, and/or as a cosmetic agent, preferably to achieve a cosmetic effect by application to a person's skin, hair, or nails.

8. A use of a fatty acid according to any one of claims 1-4 for cryopreservation and/or as a frost protection agent.

9. A use of a fatty acid according to any one of claims 1-4 for the cryopreservation of animal cells, in particular human cells, especially stem cells, muscle cells, blood cells, nerve cells, skin cells, sperm cells and/or egg cells, of tissues, in particular tissues of the cardiovascular system, the respiratory system, the digestive system, the urinary system, the musculoskeletal system and/or the nervous system, of embryos, and/or of organs, in particular heart, kidney, intestine, lungs, skin, pancreas, and/or liver.

10. A use of a fatty acid according to any one of claims 1-4 for the cryopreservation of prokaryotic cells, in particular bacteria and archaea, plant cells and/or plant tissues, in particular plant seeds, tubers, bulbs, rhizomes, and/or germ buds.

## Revendications

1. Acide gras selon la formule I : 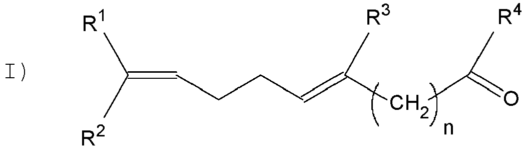
où n est supérieur ou égal à 4,
R¹ = (CH₂)ₖ-CH₃ avec k = 0, 1, 2 ou 3,
R² = (CH₂)ₘ-CH₃ avec m = 0, 1, 2, 3 ou 4,
R³ = hydrogène ou (CH₂)ₚ-CH₃ avec p = 0, 1, 2, 3 ou 4
et
R⁴ = OH, SH, NH, coenzyme A (thioester), phosphatidylcholine, phosphatidyléthanolamine, phosphatidylglycérol, phosphatidylsérine, phosphatidylinositol, sphingobase, alkyle en C₁-C₁₀ substitué ou non substitué, insaturé ou saturé, ramifié ou non ramifié, un radical de formule II : 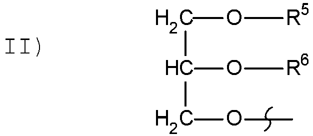 ou un radical de formule III : 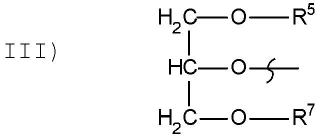
où R⁵ = hydrogène, phosphatidylcholine, phosphatidyléthanolamine, phosphatidylglycérol, phosphatidylsérine, phosphatidylinositol ou alkylcarbonyle en C₂-C₂₄ saturé ou insaturé,
R⁶ = hydrogène, alkylcarbonyle en C₂-C₂₄ saturé ou insaturé,
R⁷ = hydrogène, phosphatidylcholine, phosphatidyléthanolamine, phosphatidylglycérol, phosphatidylsérine, phosphatidylinositol ou alkylcarbonyle en C₂-C₂₄ saturé ou insaturé,

2. Acide gras selon la revendication 1, **caractérisé en ce que** les deux doubles liaisons interrompues par deux groupes méthylène se présentent dans la configuration cis.

3. Acide gras selon la revendication 1 ou 2, **caractérisé en ce que**, dans la formule I), k = 0, m = 1 et n= 4, 5, 6, 7, 8, 9 ou 10.

4. Acide gras selon l'une des revendications précédentes, **caractérisé en ce que** l'acide gras est un acide anteisoheptadéca-9,13-diénoïque, et que, dans la formule I), k = 0, m = 1, n = 7, R³ = hydrogène et R⁴ = OH.

5. Procédé d'obtention d'un acide gras selon l'une des revendications 1 à 4 par extraction de lipides de bactéries de la famille des *Flavobactericeae.*

6. Procédé d'obtention d'un acide gras selon la revendication précédente, **caractérisé en ce que** les bactéries appartiennent au genre *Chryseobacterium* et sont de préférence des *Chryseobacteria frigidisoli* et de manière tout à fait préférée des *Chryseobacteria frigidisoli* de la souche PB4^{T}.

7. Utilisation d'un acide gras selon l'une des revendications 1 à 4 comme complément alimentaire de préférence pour améliorer la capacité de concentration, la circulation sanguine et/ou le bien-être et/ou pour réduire l'insomnie, la nausée et/ou les problèmes digestifs d'une personne et/ou comme agent cosmétique, de préférence pour obtenir un effet cosmétique par application sur la peau, les cheveux ou les ongles d'une personne.

8. Utilisation d'un acide gras selon l'une des revendications 1 à 4 pour la cryoconservation et/ou comme agent anti-gel.

9. Utilisation d'un acide gras selon l'une des revendications 1 à 4 pour la cryoconservation de cellules animales, en particulier humaines, notamment de cellules souches, de cellules musculaires, de cellules sanguines, de cellules nerveuses, de cellules cutanées, de spermatozoïdes et/ou d'ovules, de tissus, en particulier de tissus du système cardio-vasculaire, du système respiratoire, du système digestif, du système urinaire, de l'appareil locomoteur et/ou du système nerveux, d'embryons et/ou d'organes, en particulier du cœur, des reins, des intestins, des poumons, de la peau, du pancréas et/ou du foie.

10. Utilisation d'un acide gras selon l'une des revendications 1 à 4 pour la cryoconservation de cellules procaryotes, en particulier de bactéries et d'archées, de cellules végétales et/ou de tissus végétaux, en particulier de graines, de tubercules, de bulbes, de rhizomes et/ou de plantules de plantes.
